**Europäisches Patentamt**

**European Patent Office**

**Office européen des brevets**

(19)

(11) Veröffentlichungsnummer: **0 083 925**
**B1**

(12) # EUROPÄISCHE PATENTSCHRIFT

(45) Veröffentlichungstag der Patentschrift:
**08.10.86**

(51) Int. Cl.⁴: **A 61 K 37/43**, A 61 K 31/56

(21) Anmeldenummer: **83100033.6**

(22) Anmeldetag: **04.01.83**

(54) **Verfahren zur Behandlung der Azyklie bei Schafen oder Rindern.**

(30) Priorität: **09.01.82 DE 3200459**

(43) Veröffentlichungstag der Anmeldung:
**20.07.83 Patentblatt 83/29**

(45) Bekanntmachung des Hinweises auf die Patenterteilung:
**08.10.86 Patentblatt 86/41**

(84) Benannte Vertragsstaaten:
**AT BE CH DE FR GB IT LI NL SE**

(56) Entgegenhaltungen:
**Keine**

(73) Patentinhaber: **HOECHST AKTIENGESELLSCHAFT,
Postfach 80 03 20, D-6230 Frankfurt am Main 80 (DE)**

(72) Erfinder: **Humke, Rainer, Dr., Amselweg 5,
D-6233 Kelkheim (Taunus) (DE)**

## Beschreibung

Die Azyklie von Rind und Schaf ist gekennzeichnet durch das Fehlen zyklischer Vorgänge am weiblichen Geschlechtsapparat und zyklusgebundener verhaltensmässiger Erscheinungen. Dieser Zustand geht von hormonellen Störungen der Achse – Hypothalamus – Hypophyse – Eierstock aus, als deren Folge die Eierstocktätigkeit ruht.

Beim Rind tritt die Azyklie bei Jungtieren auf, die zum ersten Mal gedeckt oder besamt werden sollen sowie bei Kühen während eines unterschiedlich langen Zeitraumes nach einer Geburt. Besonders ausgeprägt ist die Azyklie nach der Geburt bei Kühen aus Fleischrassen sowie bei Muttertieren, die ein oder mehrere Kälber säugen. Da die Wirtschaftlichkeit eines Muttertieres von einer regelmässigen Abkalbung – möglichst im Abstand von 1 Jahr – bestimmt wird, besteht seitens des Tierhalters grösstes Interesse, den Zeitraum der Azyklie so kurz wie möglich zu halten.

Das Schaf ist von Natur aus saisonal polyöstrisch, d.h. eine Brunst tritt in regelmässigen Abständen nur während einer begrenzten Jahreszeit auf. Nach erfolgter Konzeption und Ablammung kommt es bis zum Beginn der nächsten Brunstsaison zu einer mehrmonatigen Azykliephase. Die Rentabilität der Schafhaltung könnte deutlich verbessert werden, wenn es gelänge, die Mutterschafe während dieser Azykliephase künstlich in Brunst und zur Konzeption zu bringen und damit die Reproduktionsrate eines Jahres praktisch zu verdoppeln.

Seit langem werden Versuche unternommen, die Azyklie bei Rind und Schaf durch Anwendung verschiedener Hormone – einzeln oder in Kombination verabreicht – zu beheben. Dabei wurden vor allem Gestagene (Progesteron und synthetische Derivate), z.T. in Kombination mit Östrogenen und PMSG (Pregnant mare's serum gonadotropin) in verschiedenen Zubereitungsformen durch Injektion, Implantation sowie bei oraler oder vaginaler Anwendung über einen mehrtägigen Zeitraum verabfolgt (Rind: J. Anim. Sci., 19., 674–677 (1960); J. Anim. Sci., 25., 497–503 (1966); Tierärztl. Umschau, 26; 1–4 (1971); Vet. Rec., 101., 13–14 (1977); Vet. Rec., 104., 523–525 (1979); Vet. Rec. 104., 603–604 (1979); J. Reprod. Fert., 53., 289–296 (1978); J. Reprod. Fert., 54., 447–458 (1978); Theriogenology, 10., 307–312 (1978). Schaf: J. Endocrinol., 24., 33 (1962); J. Anim. Sci., 34., 1011–1019 (1972); Res. in Vet. Sci., 22., 324–329 (1977); J. Reprod. Fert., 44., 59–68 (1975); VIIIth Intern. Congr. Anim. Reprod. Krakow, 62 (1976); Theriogenology, 15., 389–403 (1981)).

Abhängig von verschiedenen Faktoren, insbesondere von den jeweiligen Rassen und vom Zeitpunkt der Behandlung nach der letzten Geburt waren die Behandlungsergebnisse recht unterschiedlich und überwiegend unbefriedigend. Die weitere Suche nach verbesserten Methoden, die unter Berücksichtigung der natürlichen hormonellen Steuerung zu besseren Ergebnissen führen könnten, ist deshalb verständlich.

Seit wenigen Jahren ist nun bei verschiedenen Spezies nachgewiesen, dass es vor und während eines Menstrual- oder Brunstzyklus zu pulsatilen Sekretionen des in der Hypophyse gebildeten Luteinisierenden (LH) sowie Follikelstimulierenden Mormones (FSH) kommt, die offenbar von pulsatilen Sekretionen des LH/FSH-Releasinghormons hervorgerufen werden. Entsprechende Untersuchungen an Schaf und Rind werden von Yuthasastrakol et al. (J. Reprod. Fert., 50., 319–321, 1977); Baird (Biology of Reproduction, 18., 359–364, 1978) sowie Carruthers et al. (J. Anim. Sci., 50., 919–925, 1980), Forrest et al. (Biol. Reprod., 22., 197–201, 1980), Peters et al. (J. Reprod. Fert., 62., 567–573, 1981) berichtet. Es lag nahe, die pulsatilen Sekretionen des LH und FSH bei azyklischen Tieren durch eine pulsatile Gabe von LH-RH zu simulieren. Diesbezügliche Versuche am Schaf werden von McLeod und Haresign (Proc. Annual Conference Soc. for the Study of Fertility Abstr., 6., 15, 1980) beschrieben. Mit intravenöser Injektion von 250, 500 oder 1000 ng LH-RH im Abstand von 2 Stunden über einen Zeitraum von 8 Tagen konnten pulsatile LH-Freisetzungen gemessen werden, und es kam dosisabhängig im Zeitabstand von 40–60 Stunden nach Beginn der Behandlung zu einem starken LH-Anstieg, wie er vor jedem Eisprung gemessen werden kann. Nach erfolgter Ovulation war der Anstieg der Progesteronwerte über einen Zeitraum von 10–12 Tagen festzustellen, welcher mit der Entwicklung eines Gelbkörpers einhergeht. Eine Brunst war mit diesem Behandlungsverfahren jedoch nicht zu induzieren, so dass es nicht möglich war, die behandelten Schafe natürlich zu paaren. Auch erwies es sich, dass die Gelbkörperfunktion in den meisten Fällen nicht der eines normalen Gelbkörpers während eines Zyklus entsprach.

Erst nach einer vierzehntägigen Vorbehandlung von 5 Schafen mit einem subkutan verabreichten, Progesteron enthaltenden Implantat und einer anschliessenden 48-stündigen Gabe von 250 ng LH-RH im Abstand von 2 Stunden konnten Brunst und Ovulationen ausgelöst werden, und 3 von 5 gepaarten Schafen wurden tragend (McLeod und Haresign, Proc. Annual Conference Soc. for the Study of Fertility, Abstr., 6., 13, 1981).

Pulsatile LH-RH Behandlungen in 1–2 stündigen Abständen wurden auch bei anderen Spezies angewandt und führten ebenfalls zu entsprechenden pulsatilen LH-Freisetzungen und dem Anlaufen eines ovariellen Zyklus (Mensch: Crowley et al., J. Clin. Endocrinol. Metab., 51., 173–175, 1980; Leyendecker et al., 61st Ann. Endocrine Soc. Meeting, Anaheim, California, Abstr., 926, 1979; Rind: Riley et al., 73rd Ann. Meeting Soc. Anim. Sci., North Carolina State University, Raleigh, Abstr., 1981; Affe: Knobil, Rec. Progr. Horm. Research, 36., 53–88, 1980).

Versuche beim Affen, mit kontinuierlicher intravenöser LH-RH Gabe die gleiche pulsatile LH-Freisetzung mit Zyklus- und Ovulationsinduktion zu bewirken, schlugen dagegen fehl (Knobil, Rec. Progr. Horm. Research, 36., 53–88, 1980). Es war somit zu erwarten, dass auch bei anderen Spezies mit kontinuierlicher Verabreichung von LH-RH nicht die gleichen Effekte zu erzielen waren wie mit pulsatiler Gabe. Andererseits ist eine pulsatile Verabreichung von Wirkstoffen beim landwirtschaftlichen Nutztier zu zeit- und kostenaufwendig und daher aus wirtschaftlichen Gründen nicht akzeptabel.

Es wurde nun gefunden, dass es beim azyklischen Schaf und Rind nach 4 bis 16tägiger Verabreichung eines Gestagens und einer nachfolgenden kontinuierlichen i.v. Gabe von etwa 250 ng LH-RH/Stunde über einen Zeitraum von 2 bis 6 Tagen zu einer pulsatilen LH-Freisetzung im Plasma, dem Eintritt einer Brunst sowie einer Auslösung von Ovulationen kam, die nach Bedeckung der Tiere in einer Trächtigkeit resultierten.

Gegenstand der Erfindung ist ein Verfahren zur Beseitigung der physiologischen Acyclie bei Schafen oder Rindern, dadurch gekennzeichnet, dass man ein Schaf oder Rind 4 bis 16, vorzugsweise 7 bis 12 Tage lang mit einem protrahiert wirksamen Gestagen behandelt und im Anschluss an diese Behandlung 2 bis 6, vorzugsweise 2 bis 3 Tage durch Gaben von LH-RH oder einem mindestens ebenso stark wirksamen Analogen einen zur Induzierung der Ovulation ausreichenden LH-Plasma-Spiegel erzeugt. Die Dosis des protrahierten wirksamen Gestagens wird so gewählt, dass während der Behandlungsdauer ein Blutspiegel induziert wird, der ausreicht, die LH- und FSH-Freisetzung aus der Hypophyse zu hemmen. (vgl. z.B. Endocrinology 108 [1981] 568–572, J. Reprod. Fert. 60 [1980] 177–185 und Res. Vet. Sci. 22 [1977] 324–329).

Neben LH-RH können für den erfindungsgemässen Zweck auch LH-RH-Analoga herangezogen werden, die die Ausschüttung von LH und FSH bewirken. Vor allem Verbindungen mit einer stärkeren biologischen Wirkung verbunden mit einer längeren Wirkungsdauer sind dazu geeignet. Infrage kommen insbesondere LH-RH-Analoga, die in Position 6 eine D-Aminosäure, in Position 7 N-Methyl-leucin und/oder in Position 10 Äthylamin, Cyclo-propylamin oder Semicarbazid enthalten.

Als D-Aminosäuren kommen vor allem lipophile Aminosäuren infrage. Besonders geeignet erwiesen sich z.B. D-Leu, D-Trp, D-Phe, D-Ser(Bu$^t$), D-His(Bzl), D-Naphthylalanin D-Glu(OBu$^t$) und D-Aad(OBu$^t$).

Die Aufrechterhaltung des LH-Plasmaspiegels mit LH-RH und den genannten LH-RH-Analoga über den genannten Zeitraum wird mit Hilfe von verzögernd wirkenden Applikationstechniken und Arzneiformen erreicht. Neben Infusionen, Injektionen, transdermaler Applikation oder Applikation auf Schleimhäuten, z.B. die der Nase, mit den ihnen zugrunde liegenden bekannten Freigabe-Prinzipien können auch implantierbare Arzneiformen wie Depotkörper oder Pumpen, intravaginale oder intrauterine Freigabesysteme oder Nasenspangen eingesetzt werden. Bei transdermaler Applikation und Applikation auf die Schleimhäute muss erfahrungsgemäss um den Faktor 10–50 mal höher dosiert werden.

Die in der ersten Phase des erfindungsgemässen Verfahrens angewandte 4 bis 16 tägige Gestagen-Behandlung kann ebenfalls mit einem Implantat, jedoch auch mit Hilfe der vorstehend erwähnten Applikationstechniken und Arzneiformen zur retardierten Gestagen-Freigabe in entsprechender Weise ausgeführt werden.

Die Gestagenvorbehandlung des in der Regel 40–75 kg schweren Schafes kann beispielsweise durch parenterale Gabe von 5–25 mg vorzugsweise ca. 10 mg Progesteron/Tag erfolgen. Weiter ist es möglich, subcutane Implantate (z.B. Sil-Estrus® der Fa. Abbott oder die in J. Reprod. Fert. 60 [1980] 177–185 beschriebenen Implantate) mit einem Gehalt von 100–500 mg Progesteron während der Dauer von 6 bis 14 Tagen einzupflanzen. Norgestomet-Ohrimplantate (Fa. Searle) enthalten ca. 3 mg Norgestomet und werden auch bis zu 14 Tage eingepflanzt. Bei der intravaginalen Applikation verwendet man beispielsweise Schwämmchen, die 10–50 mg, vorzugsweise 20–40 mg Fluorogestonacetat enthalten, während der Dauer von bis zu 14 Tagen (z.B. Syncro-Mate® Schwämmchen mit Cronolone der Fa. Searle). Die orale Behandlung mit MAP wird beispielsweise in J. Animan Sci., Albany, N.Y. 48 [1979] 1015–1019 beschrieben.

Die Gestagenvorbehandlung des in der Regel 400–750 kg schweren Rindes kann beispielsweise durch subcutane Injektion von 20–200 mg vorzugsweise ca. 50 mg Progesteron/Tag während etwa 10 Tagen erfolgen. Beispielsweise können auch ca. 120 mg Dihydroprogesteron-acetophenid/Tag oral etwa 10 Tage lang gegeben werden. Das wirksamere 19-Nortestosteron braucht oral in täglichen Dosen von nur ca. 10 mg appliziert werden. In der gleichen Grössenordnung liegen die Dosen bei Chlormadinonacetat (vgl. Berl. Münch. Tierärztl. Wschr. 86 [1973] 384–387). Synchro-Mate® Ohrimplantate enthalten etwa 6 mg Norgestomet und verbleiben bis zu 9 Tage im Tier. Die Intravaginal-Devices PRID® (Fa. Abbott) enthalten bis zu 1,6 g Progesteron und werden in der Regel 7–9 Tage angewandt.

Die LH-RH-Behandlung kann beispielsweise wie nachstehend beschrieben erfolgen:

Die Gaben von LH-RH oder dessen Analogen betragen auf LH-RH berechnet beim Schaf in der Regel 20–1000 vorzugsweise 50–500 ng/h (bei i.v.-Applikation oder bei der Infusion). Subcutan implantierte Depotkörper enthalten bis zu 500 µg vorzugsweise 10–100 µg LH-RH oder die entsprechende Menge eines Analogen (Verweildauer 2 bis 6 Tage).

Beim Rind werden in der Regel 40–2000 vorzugsweise 120–1000 ng LH-RH/h (oder die entsprechende Menge eines Analogen) i.v. oder durch Infusion appliziert. Subcutan implantierte Depotkörper enthalten bis zu 6000 µg vorzugsweise 150–1200 µg LH-RH oder die entsprechende Menge eines Analogen (Verweildauer 2 bis 6 Tage).

### Beispiel 1

28 weiblichen geschlechtsreifen Schafen zweier verschiedener Rassen («Clun Forest» und «Welsh Halfbred») wurden über einen Zeitraum von 14 Tagen subkutan Progesteron enthaltende Implantate verabreicht.

2 Stunden vor Herausnahme der Implantate erhielten jeweils 12 Schafe über einen Zeitraum von 48 Stunden eine Dauerinfusion, mit der 125 bis 250 ng LH-RH pro Stunde appliziert wurden. 4 entsprechend mit einer Kochsalzlösung behandelte Schafe dienten als Kontrolle. Zur Überprüfung der Ovulationen wurden die Schafe laparoskopiert; ausserdem wurden Blutproben zur Progesteronbestimmung entnommen. 24 Stunden nach Beginn der Infusionen wurden ferner in 4-stündlichem Abstand mit fruchtbaren Schafböcken der Brunsteintritt überprüft und die brünstigen Schafe gedeckt.

Die Ergebnisse sind in nachfolgender Tabelle zusammengefasst.

| Behandlung | Anzahl gedeckter Schafe | Anzahl Ovulationen | Ovulationsrate |
|---|---|---|---|
| 125 ng LH-RH pro Stunde | 10* | 9** | 1,4 |
| 250 ng LH-RH pro Stunde | 11 | 12 | 1,5 |
| physiolog. NaCl Lösung | 0 | 0 | 0 |

*  Ein Schaf hatte das Progesteronimplantat vor der LH-RH-Behandlung verloren und konnte somit nicht brünstig werden.
** Ein Schaf konnte wegen innerer Verwachsungen nicht laparoskopiert werden.

Auf Grund der zum Berichtszeitpunkt vorliegenden Progesteronwerte von 12 mit LH-RH behandelten Schafen sind 9 Schafe als tragend anzusehen.

Die damit erzielte Trächtigkeitsrate von 75% entspricht dem während der natürlichen Paarungssaison erreichbaren Durchschnittswert.

Beispiel 2

17 Kühen von Fleischrinderrassen wurde in den ersten Wochen nach der Geburt über einen Zeitraum von 2–4 Tagen im Abstand von 2 Stunden jeweils 1,0; 2,5 bzw. 5,0 µg LH-RH injiziert. 21 entsprechende Kühe erhielten während des gleichen Zeitraums eine Dauerinfusion mit den gleichen Dosierungen pro Zeiteinheit.

Vor Beginn sowie während der Behandlungen wurden Blutproben zur Bestimmung von LH entnommen. Blutentnahmen zur Bestimmung von Progesteron erfolgten über einen längeren Zeitraum nach der Behandlung.

Die Ergebnisse der Progesteronbestimmung sind der nachfolgenden Tabelle zu entnehmen:

Tabelle

| Behandlung | Dosis (µg) | Anzahl der Kühe | |
|---|---|---|---|
| | | gesamt | davon mit Progesteron- anstieg |
| Injektionen in Abständen von 2 Stunden | 1,0 | 6 | 4 |
| | 2,5 | 6 | 4 |
| | 5,0 | 5 | 4 |
| Dauerinfusion (µg/2 Stunden) | 1,0 | 6+3 | 2 |
| | 2,5 | 6 | 4 |
| | 5,0 | 6 | 4 |

Bei den in zweistündigem Abstand mit Dosierungen von jeweils 1,0; 2,5 bzw. 5,0 µg injizierten Kühen kam es bei 4 von 6, 4 von 6 bzw. 4 von 5 Tieren zu einem Anstieg des Progesteronspiegels, welcher eine zuvor erfolgte Ovulation anzeigt. Nach Dauerinfusionen mit analogen Dosierungen traten Progesteronerhöhungen bei 2 von 9, 4 von 6 bzw. 4 von 6 Tieren auf.

6 der 9 mit 1,0 µg/2 Stunden dauerinfundierten Kühen wurden nach Frühjahrskalbungen behandelt und reagierten nicht. Die drei restlichen Kühe hatten im Sommer gekalbt; von ihnen reagierten zwei Tiere.

Unabhängig von der Applikationsweise und den oben genannten Dosierungen kam es bei allen Versuchstieren während der Behandlungsphase im Vergleich zur Vorbehandlungsphase zu einem deutlichen Anstieg der LH-Konzentrationen im Plasma, wobei die durch die LH-Gaben induzierten Pulsfrequenzen den endogenen LH-Mustern während der Kontrollphase entsprachen.

Aus diesen Befunden lässt sich klar ableiten, dass mit einer LH-RH-Dauerinfusion beim Rind die gleichen pharmakologischen und klinischen Effekte erzielt werden können wie nach pulsatiler LH-RH-Gabe.

### Beispiel 3

5 Kühe von Fleischrinderrassen wurden in den ersten Wochen nach Geburt über einen Zeitraum von 8 Tagen mit einer Progesteron enthaltenden Intravaginalspirale (PRID® /Fa. Abbott) behandelt. Nach Herausnehmen der Spirale erfolgte eine wie im Beispiel 2 beschriebene LH-RH-Dauerinfusion mit 2,5 µg LH-RH/2 Stunden. Bei allen 5 Kühen kam es während der Behandlung zu einer Ovulation, die durch den Anstieg des Progesteron-Plasmaspiegels gekennzeichnet war.

### Patentansprüche

1. Verfahren zur Beseitigung der physiologischen Azyklie bei Schafen oder Rindern, dadurch gekennzeichnet, dass man ein Schaf oder Rind 4 bis 16 Tage lang mit einem protrahiert wirksamen Gestagen behandelt und im Anschluss an diese Behandlung 2 bis 6 Tage durch kontinuierliche Gaben von LH-RH oder einem mindestens ebenso stark wirksamen Analogen einen zur Induzierung der Ovulation ausreichenden LH-Plasma-Spiegel erzeugt.

2. Verwendung von Gestagenen und LH-RH oder dessen Analogen bei der Beseitigung der physiologischen Azyklie bei Schafen oder Rindern durch 4–16-tägige Anwendung eines protrahiert wirkenden Gestagens und anschliessende 2–6-tägige kontinuierliche Verabreichung von LH-RH oder einem mindestens ebenso stark wirksamen Analogen in einer Dosis, die einen zur Induzierung der Ovulation ausreichenden LH-Plasmaspiegel erzeugt.

3. Erzeugnis enthaltend
a) ein Gestagen in Form einer Zubereitung zur retardierten Freigabe und
b) LH-RH oder ein mindestens ebenso stark wirksames Analoges in Form einer Zubereitung zur kontinuierlichen Verabreichung als Kombinationspräparat zur zeitlich abgestuften Anwendung bei der Behandlung der pathologischen Azyklie bei Schafen und Rindern.

4. Verfahren gemäss Anspruch 1, dadurch gekennzeichnet, dass man LH-RH-Analoga verwendet, die in Position 6 als D-Aminosäure D-Ser(Buᵗ) enthalten.

5. Verfahren gemäss einem der Ansprüche 1 oder 4, dadurch gekennzeichnet, dass man LH-RH-Analoga verwendet, die in Position 10 Äthylamin enthalten.

6. Verwendung von LH-RH-Analoga gemäss Anspruch 2, dadurch gekennzeichnet, dass diese in Position 6 als D-Aminosäure D-Ser(Buᵗ) enthalten.

7. Verwendung von LH-RH Analoga gemäss einem der Ansprüche 2 oder 6, dadurch gekennzeichnet, dass diese in Position 10 Äthylamin enthalten.

8. Erzeugnis gemäss Anspruch 3, dadurch gekennzeichnet, dass es ein LH-RH Analoges mit der Aminosäure D-Ser(Buᵗ) in Position 6 enthält.

9. Erzeugnis gemäss einem der Ansprüche 3 oder 8, dadurch gekennzeichnet, dass es ein LH-RH Analoges mit Äthylamin in Position 10 enthält.

### Claims

1. Process for eliminating physiological anestrus in ewes or bovine animals which comprises treating a ewe or bovine animal for 4 to 16 days with a gestagen having a protacted action and, following this treatment, administering LH-RH or an analogous compound thereof having at least the same efficiency for 2 to 6 days to bring about the LH plasma level sufficient to induce ovulation.

2. Use of gestagens and LH-RH or analogous compounds thereof in the elimination of physiological anestrus in ewes or bovine animals by administering a gestagen having a protacted action for 4 to 16 days and by administering subsequently LH-RH or an analogous compound thereof having at least the same efficiency for 2 to 6 days in doses bringing about a LH plasma level sufficient to induce ovulation.

3. Product comprising
a) a gestagen in form of a preparation for retarded release and
b) LH-RH or an analogous compound having at least the same efficiency in form of a preparation for administering it as a combined preparation for temporally graduated application by treating pathological anestrus in ewes and bovine animals.

4. Process as claimed in claim 1 which comprises using LH-RH analogous compounds having D-Ser(Buᵗ) in position 6 as D-amino acid.

5. Process as claimed in one of claims 1 or 4 which comprises using LH-RH analogous compounds having ethylamine in position 10.

6. Use of LH-RH analogous compounds as claimed in claim 2 having D-Ser(Buᵗ) in position 6 as D-amino acid.

7. Use of LH-RH analogous compounds as claimed in one of claims 2 or 6 having ethylamine in position 10.

8. Product as claimed in claim 3 which comprises a LH-RH analogous compound having the amino acid D-Ser(Buᵗ) in position 6.

9. Product as claimed in one of claims 3 or 8 which comprises a LH-RH analogous compound having ethylamine in position 10.

### Revendications

1. Procédé pour remédier à l'absence physiologique de cycle (acyclie) chez les ovidés ou les bovins, caractérisé en ce qu'on traite un ovidé ou un bovin pendant de 4 à 16 jours avec un gestagène à effet retard et à la fin de ce traitement on administre en continu pendant de 2 à 6 jours de la LH-RH ou un analogue au moins aussi actif, de manière à produire un taux palsmatique de LH suffisant pour induire l'ovulation.

2. Utilisation de gestagènes et de LH-RH ou de ses analogues pour remédier à l'absence physiologique de cycle chez les ovidés ou les bovins par application pendant de 4 à 16 jours d'un gestagène à action retard, puis administration continue pendant de 2 à 6 jours de LH-RH ou d'un analogue au moins aussi actif, en une dose qui produit un taux plasmatique de LH suffisant pour induire l'ovulation.

3. Produit contenant:

a) un gestagène sous la forme d'une préparation pour libération retardée, et

b) LH-RH ou un analogue au moins aussi actif sous forme d'une préparation pour une administration continue comme préparation combinée pour une utilisation graduelle dans le temps lors du traitement de l'absence physiologique de cycle chez les ovidés et les bovins.

4. Procédé selon la revendication 1, caractérisé en ce qu'on utilise des analogues de LH-RH qui contiennent en position 6 D-Ser(Bu$^t$) en tant qu'acide D-aminé.

5. Procédé selon l'une quelconque des revendications 1 à 4, caractérisé en ce qu'on utilise des analogues de LH-RH qui contiennent en position 10 un groupe éthylamine.

6. Utilisation d'analogues de LH-RH selon la revendication 2, caractérisé en ce qu'ils contiennent en position 6 D-Ser(Bu$^t$) en tant qu'acide D-aminé.

7. Utilisation d'analogues de LH-RH selon l'une des revendications 2 et 6, caractérisés en ce qu'ils contiennent en position 10 un groupe éthylamine.

8. Produit selon la revendication 3, caractérisé en ce qu'il contient un analogue de LH-RH avec D-Ser(Bu$^t$) en position 6 en tant qu'acide D-aminé.

9. Produit selon l'une des revendications 3 et 8, caractérisé en ce qu'il contient un analogue de LH-RH avec un groupe éthylamine en position 10.